# EUROPEAN PATENT SPECIFICATION

(11) **EP 1 561 442 B1**
(45) Date of publication and mention of the grant of the patent: **30.06.2010**
(21) Application number: 05250695.3
(22) Date of filing: 08.02.2005
(51) Int. Cl.: A61F 13/15

(54) **Fluid management article and methods of use thereof**
Flüssigkeitsbehandlungssystem und seine Nutzungsmethode
Gestion de fluid et methode d'utilisation

(30) Priority: 09.02.2004 US 543005 P; 19.01.2005 US 39240 P
(43) Date of publication of application: 10.08.2005
(73) Proprietor: McNeil-PPC, Inc., Skillman, New Jersey 08558 (US)
(72) Inventor: Berba, Maria Luisa M., New Manila Quezon City 1112 (PH); Ren, Joseph, 201100 Shanghai (CN); Marcelo, Ana Maria R., Antipolo Rizal 1870 (PH); Collado, Santos, Las Pina City 1740 (PH)
(74) Representative: Mercer, Christopher Paul

(56) References cited:
- EP-A- 0 336 578
- EP-A- 0 705 584
- EP-A- 0 719 531
- EP-A- 1 086 677
- US-A- 4 500 315
- US-A- 5 803 920
- US-A- 6 103 953
- US-B1- 6 231 556

## Description

### Field of the Invention

The present invention relates to a thin fluid management article that combines bulkiness with limited fluid absorbency to achieve a soft, comfortable article suitable for daily management of body exudates.

### Background of the Invention

Fluid management articles may be disposable absorbent articles, such as, pantiliners, sanitary napkins, interlabial devices, adult incontinence devices, bandages, breast pads, and diapers. These articles typically have a fluid permeable body-facing side and fluid impermeable garment facing side and may include a layer for holding body exudates, e.g., an absorbent core or non-absorbent core, there between. Such articles have traditionally been made from readily available and relatively inexpensive materials, such as, absorbent materials, e.g., cotton fibers, wood pulp fluff, cellulosic tissue, or wadding, or non-absorbent materials, e.g., foam.

Products that have become popular with consumers include those known as ultrathins. Ultrathin products are typically used during menstruation and not for daily management of body exudates. While ultrathin products absorb the same amount or a greater amount of body exudates as a standard sanitary napkin, the caliper, i.e., thickness, of ultrathin products is typically less than standard sanitary napkins. For example, the caliper of a typical ultrathin is about 1.5 mm. Such thinness is achieved in ultrathin products by the use of superabsorbents, including superabsorbent polymers (SAP).

Recently, it has also been found that more and more consumers are wearing absorbent articles on a daily basis and, therefore, do not require the fluid absorption found in ultrathins or in standard sanitary napkins. There is a need in the market for a thin absorbent article that is comfortable and discrete while having nominal fluid management.

U.S. Pat. No. 6,440,111 discloses a fluid management article that, in one embodiment, is absorbent free and capable of collecting fluid within existing interstitial spaces or pores. The spaces or pores are formed between non-absorbent fibers or within apertures or bosses. In another embodiment, the article has a cover, a barrier layer and an absorbent core intermediate the cover and barrier. The core contains limited amounts of absorbent material, e.g, 0.7 g. or less. Such articles were found to have a caliper of 3 millimeters or less and a flexural resistance of 120 grams or less. The low total capacity of this embodiment, i.e., 1.2 grams or less, limits the usefulness of this article.

What is lacking in the marketplace is a fluid management article that provides comfort and discretion for every day use having increased absorption properties coupled with a thin, flexible structure.

According to the present invention there is provided a fluid management article as defined in the appendant claims.

### Brief Description of the Drawings

FIG. 1 is a top plan view of an absorbent article according to the invention;
FIG. 2 is a cross-sectional view taken across line A-A of the embodiment shown in FIG. 1;
FIG. 3 is a top plan view of one embodiment of an absorbent article according to the invention; and
FIG. 4 is a cross-sectional view taken across line B-B of the embodiment shown in FIG. 3.

### Detailed Description of the Invention

The fluid management article of the present invention has a bulk that contributes to softness and comfort but has the caliper of an ultrathin and the absorbent capacity to ensure protection during or between menstrual cycles, e.g. for daily use.

As used herein, the term "fluid management article" shall mean a disposable absorbent article, such as, a pantiliner, sanitary napkin, adult incontinence device, breast pad, and, bandage. These articles typically have a fluid permeable body-facing side and fluid impermeable garment facing side. Additionally, such articles may include an absorbent or non-absorbent core for fluid retention therebetween.

As used herein, the term "absorbent" shall mean any material or composite that absorbs fluid, especially body exudates.

As used herein, the term "fluid retention" shall mean the ability of a material to retain and hold fluid. Absorption of the fluid by the material is not necessary for the material to be considered to have fluid retention. Fluid retention may be accomplished merely by holding fluid within voids or spaces between fibers, within apertures, depression, such as, embossments, and the like.

As used herein, term "SAP" means particles or fibers that are capable of absorbing many times, at least 10, more preferably 15, and still more preferably over 15, their weight in fluids or bodily exudates, under a pressure of 0.5 psi.

As used herein, the phrase "weight percent" means weight of substance per weight of final material as determined under ambient conditions. By way of nonlimiting example, 10 weight percent absorbent cellulose-based material means 10 g/m² cellulose-based material per 100 g/m² basis weight of total material.

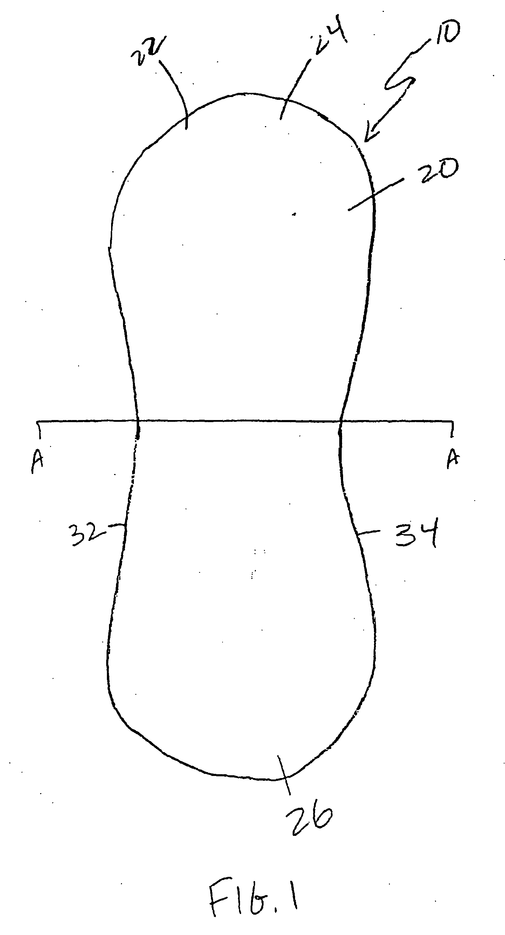

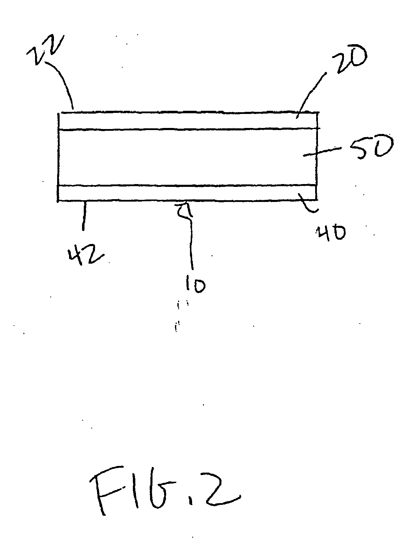

The present invention relates to a fluid management article designed and configured to be worn adjacent a user's perineum, which is useful for collecting and/or absorbing low volumes of bodily fluids. As seen in Figures 1 and 2, fluid management article 10 may be any desired configuration in terms of shape and construction; but, generally speaking, it includes a cover 20 having body facing surface 22, a backsheet or barrier 40 having a garment facing surface 42, and a unitized core structure 50 . The fluid management article 10 has a first end portion 24 in opposed to a second end portion 26 and a first longitudinal edge 32 opposed to a second longitudinal edge 34, wherein the edges connect the two opposed end portions.

Referring to FIG. 2, in this embodiment, the fluid management article includes a cover 20 having body facing surface 22 and a barrier 40 having a garment facing surface. The cover 20 may have the following, non-limiting configurations, a nonwoven web, a woven web, an apertured film, an apertured formed film, a substrate having flocked fibers thereon, a lamination of multiple layers of films or fibrous webs and combinations thereof, or the like. Unitary core structure 50, which is between cover 20 and barrier 40, may contain low amounts of absorbent material dispersed within the non-absorbent material.

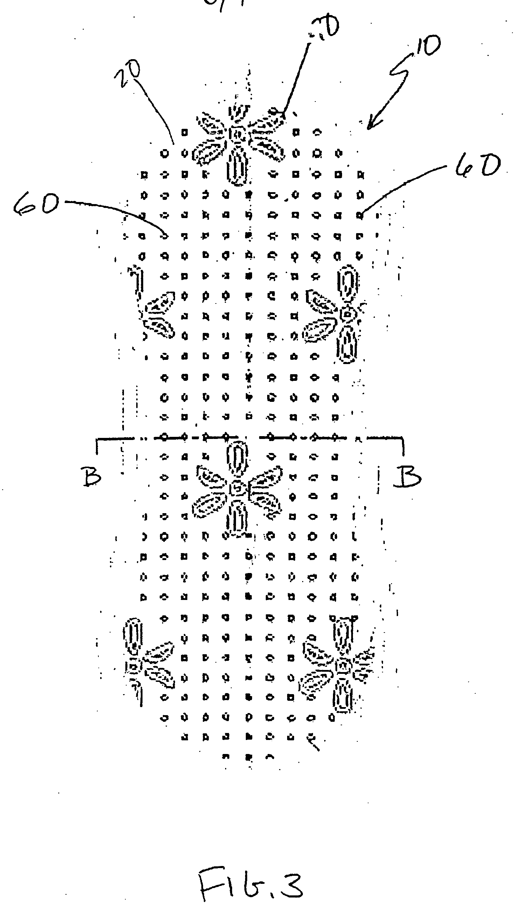

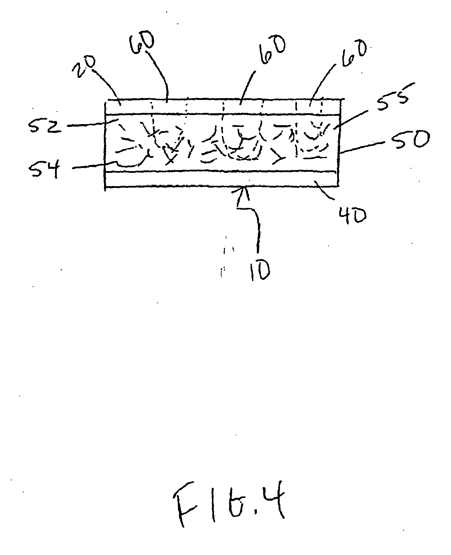

In one particular embodiment of this invention, the fluid management article 10 is shown in Figures 3 and 4. In this embodiment, the fluid management article 10 includes a cover 20 having body facing surface 22 and a barrier 40 having a garment facing surface. The cover 20 may have the following, non-limiting configurations, a nonwoven web, a woven web, an apertured film, an apertured formed film, a substrate having flocked fibers thereon, a lamination of multiple layers of films or fibrous webs and combinations thereof, or the like. Unitary core structure 50, which is between cover 20 and barrier 40, may contain low amounts of absorbent material dispersed within the non-absorbent material. The cover 20 and unitary core structure 50 are embossed together, or co-embossed, using a pattern of dots 60. In addition, this particular embodiment, a series of flowers 90 are embossed on the cover prior to co-embossing the cover and the unitary core structure 50.

### Cover

The fluid management article 10 of the present invention includes a cover layer 20 overlaying the absorbent material. The exterior of the cover forms the body-facing surface 22 of the disposable absorbent article. As known by those skilled in the art, the cover layer 20 may be formed from any fluid pervious material that is generally compliant, soft feeling, and non-irritating to the user's skin and permits fluid to penetrate to the absorbent core, which retains the fluid. The cover layer 20 generally functions to transport fluid away from the wearer into the absorbent article. In this manner, fluid and moisture are removed from contacting the wearer, thus making the wearer feel dry and comfortable. In addition to transporting fluid, the cover layer 20 may also absorb and/or retain fluid as well.

The cover 20 can be made from any of the materials conventional for this type of use. Non-limiting examples of suitable materials that can be used as the cover layer 20 are woven and nonwoven fabrics formed fibers or filaments of cellulose, polyester, polyethylene, polypropylene, nylon, rayon fibers and mixtures thereof or the cover layer may be an apertured thermo-plastic film and formed films. Other materials used in making covers layer 20 include gauze or any known porous material with a suitable body contacting surface, including, but not limited to nonwoven webs, plastic nets, and the like. The cover layer 20 could also be made from a fibrous nonwoven composite of bicomponent fibers and pulp fluff.

Bicomponent fibers are known in the art and are composed of two polymers with different melting points. At least a portion of the outer surface of each bicomponent fiber has the lower melting polymer. The two polymers may be arranged such that a cross-section of the fiber shows the two polymers in a side-by-side array. Alternatively, the polymers may be positioned in a so-called sheath/core arrangement, in which a core of higher melting polymer is surrounded by a sheath of lower melting polymer.

The cover may be made by a process of bonding a nonwoven conjugate fiber web in which heated air, which is sufficiently hot to melt one of the polymers of the multicomponent fibers, is forced through the web. This process is referred to as through air bonding. The melting and resolidification of the polymer provides the bonding between the fibers to integrate the web. The air velocity is typically between 100 and 500 feet per minute (30 and 152 m/min) and the dwell time may be as long as 6 seconds. Through air bonding has relatively restricted variability and since through-air bonding requires the melting of at least one component to accomplish bonding, it is particularly useful in connection with webs of conjugate fibers or those which include an adhesive. In the through-air bonder, air having a temperature above the melting temperature of at least one of the exposed components is directed through the web and into a perforated roller supporting the web. Alternatively, the through-air bonder may be a flat arrangement wherein the air is directed vertically downward onto the web. The operating conditions of the two configurations are similar, the primary difference being the geometry of the web during bonding.

A useful bicomponent fiber is a 2.0 denier, 45 mm long staple fiber made of a polyester core and a high density polyethylene sheath. Similar fibers (polyethylene sheath and polypropylene core) are available as Danaklon ES-C or ES Bico (Danaklon A/S, Varde Denmark) or ES Fiber GuangZhou Co. Ltd. (Chisso). Pulp fibers may be obtained as IP "'SUPERSOFT" ELM supplied by the International Paper Company (Memphis, Tennessee), "'RAYFLOC" E-Type Cellulosic Fluff Pulp, (ITT Rayonier), or Korsnas Vigorfluf-EN White (KorsncAs, Gavle, Finland). Georgia Pacific EF-100, or Weyerhauser NB416L, treated or untreated pulp.

The cover layer 20 may optionally be treated with surfactant to manipulate the hydrophobicity/hydrophilicty thereof to facilitate optimal fluid transport properties. The fibers or other materials that make up the cover layer 20 should not collapse or lose their resiliency when subjected to body fluid. The fibers may be oriented by a carding process and thermally bonded via embossing. The fiber or filament can be single denier or multidenier.

The cover may be a single layer or be made from multiple layers. The thickness of the cover may vary from about 0.001 inch (0.025 mm) to about 0.200 inch (5.000 mm), depending on the material chosen. The weight of the body facing layer material preferably is between about 5 and about 150 gsm, more preferably between 15 and 50 gsm and most preferably between 25 and 35 gsm. The cover material is preferably though air bonded. Low denier fibers are preferred to form the fiber web and the temperature of the hot air may be adjusted to bond only the material having the lower melting point. This creates a softer and loftier fiber web.

In one embodiment, the cover is a single layer made from a 2d, 45 mm bicomponent fiber made of a polypropylene core and a high density polyethylene sheath having a weight of 30 gsm.

In another embodiment, the cover is a single layer made from 1.5d, 45 mm bicomponent fiber made from polypropylene core and high density polyethylene sheath having a weight of 25 gsm.

Any material with soft or cloth-like features may also be used for the body facing layer. Such material includes nonwoven, such as spunlace, woven, and knitted materials. Optionally, the material used for the body-facing layer may include binders, such as thermoplastic binder fibers and latex binders.

In one embodiment, the cover is a single sheet of material having a width sufficient to form the body-facing surface of the absorbent article. In another embodiment, the cover has at least two layers and is made of materials described in the core section.

The cover, whether a single layer or multiple layers, may also retain fluid. If a separate core layer is used, the body facing layer may be longer and wider than the core or be of similar size as the core.

Generally, the optional cover layer 20 is a single sheet of material having a width sufficient to form the body-facing surface 22 of the fluid management article. The cover layer 20 may be longer and wider than the optional core.

The cover layer 20 may be embossed with shapes within a given area. For example, a series or a number of features, e.g., circles, triangles, squares, lines, honeycomb, diamond, floral, etc. are embossed over the entire length and width of the outer surface of web. Each embossed feature has a major and minor axis extending therethrough, the major axis length being greater or equal to the minor axis length. The embossed features may be in a repetitive pattern. The embossed pattern may contribute to the softness and drapeability of the fluid management article. The embossments may also serve to hold fluid or to transfer fluid to the core. In an embodiment shown in Figure 3, flower 90 is embossed in a pattern on cover 20.

### Unitary Core Structure

Nonwoven webs are preferred as starting materials to form the unitary core structure 50. Suitable fibers useful for making such nonwoven webs include polyolefin, polyester fibers, bicomponent fibers, and mixtures thereof. Such fibers may be fusible fibers.

In one embodiment, two materials are joined by through air bonding to form an integrally formed composite material 55 that can be used in the unitary core structure 50. In one embodiment, the composite material 55 includes a first material 52 having at least about 50% non-absorbent fusible fibers interspersed with absorbent cellulosic based fiber and a second material 54 having non-absorbent fusible fibers wherein the first and second materials are laminated through air through bonding process.

As seen in Figure 4, the fibers making up first material 52 and second material 54 are preferably of at least two different deniers, such as, 3 and 2 denier ("d") fibers, respectively. The first material 52 may contain an absorbent fiber, such as, rayon in amounts equal to or less than about 25 weight percent unitary core structure 50. In one embodiment, the first material 52 contains about 0.086 grams of 3.3d, 27 mm rayon fibers from Danufil V from Acordis Kelheim, GmbH, dispersed throughout 3d, 45 mm air through bonded bicomponent (polypropylene/polyethylene fibers) web having a weight of 25 gsm available from Guang Zhou ES Fiber Co., Ltd. The second material 54 is a 2d, 45 mm, through air bonded bicomponent fibers (polypropylene/polyethylene fibers) web having a weight of 25 gsm available from Guang Zhou ES Fiber Co., Ltd. The two materials 52 and 54 are integrated using heat to form a composite material 55 having an overall weight of 50 gsm wherein no visible distinction between materials 52 and 54, i.e, the materials 52 and 54 are integrally joined to form composite material 55. Composite material 55 has fluid retention properties.

The nonwoven webs used as materials for the composite materials have a basis weight from 20 to 200 grams per square meter ("gsm"), preferably from 25 to 50 grams per square meter. In one embodiment, the first material 52 and the second material 54 have basis weight of 25 gsm, respectively, to yield a composite material of 50 gsm.

Raw materials, such as, individual fibers, that are used in the manufacture of the unitary core structure 50, may optionally be treated with a surface active agent, e.g., a surfactant, to render the structure more hydrophilic or hydrophobic as desired.

The unitary core structure 50 of the present invention may contain any known absorbent materials including, but not limited to, absorbent fibers, such as, cellulose fibers, including, but not limited to wood pulp, regenerated cellulose fibers, e.g., rayon and cotton fibers, rayon fibers and the like; other naturally occurring absorbent materials, such as, sphagnum or peat moss; and other synthetic absorbent materials. Additionally, the unitary core structure 50 may include one or more of the following: binders, such as, thermoplastic and latex, odor-controlling compounds, e.g., perfumes, EDTA (ethylenediaminetetraacetic acid), anti-microbial agents, wetting agents, wetness indicator material, materials for administering or delivering medicaments, such as encapsulated medicaments, and materials for maintaining skin moisture, such as encapsulated moisturizers.

The core may include only materials, such as, a hot melt adhesive containing fluid absorbing polymers. One example of such a material is disclosed in EP 1 013 291 A1.

A representative, non-limiting list, of absorbent material useful in the unitary core structure 50 includes, but is not limited to, natural cellulosics, such as, cotton and wood pulp; regenerated cellulosics, such as rayon and cellulose acetate; peat moss, loofa; and the like. One skilled in the art would readily appreciate that a blend of two or more types of absorbent materials may be used to optimize the performance of fluid management articles used in varying conditions. The absorbent material may be uniformly dispersed within the unitary core structure 50, or may alternatively be placed in discrete patterns, or in gradients. For example, in an effort to help reduce side leakage, absorbent material may be placed in high concentrations around peripheral portions of the core.

The unitary core structure 50 may have a blend of absorbent materials and thermoplastic fibers, for example to provide structural integrity to the formed structure or for heat sealability to additional layers, such as a barrier layer film. Useful thermoplastic fibers are polyolefins, such as polypropylene and polyethylene fibers, and bicomponent fibers. The thermoplastic fibers may be bi-component or multi-component fibers having a first component having a first melting temperature and two or more additional components having different melting temperatures to that of the first melting temperature. Bi-component fibers are typically configured sheath-core or side-by-side. Suitable bi-component fibers include polyester/polyethylene and polypropylene/polyethylene.

In one aspect, the present invention can provide a fluid management article containing at most 12 weight percent of an absorbent material such as cellulose-based material.

As previously stated, the cover 20 and unitary core structure 50 may be joined together by co-embossing. The dots 60 will provide additional fluid retaining spaces and may contribute to the overall softness in terms compressibility. One can calculate compressibility as follows: thickness of uncompressed material minus the thickness of the compressed material (2.5 pounds per square inch) multiplied by 100 and dividing the product by the thickness of the uncompressed material. The dots 60 may also aid in providing flexibility to the finished article; the embossed dots allow the article to bend without surface deformation or wrinkling.

### Barrier

The barrier, also called backsheet 40, may be located adjacent to core 50 (shown Figures 2 and 4) and also to the cover 20, especially if the absorbent core is smaller than the cover and barrier (not shown). The barrier 40 of the present invention is a body fluid impervious material, which is at least substantially impermeable to liquids. Its exterior forms the garment-facing surface of the absorbent article. The barrier 40 may be any thin, flexible, body-fluid impermeable material, such as, but not limited to, a polymeric film, e.g., polyethylene, polypropylene, or cellophane, or a normally fluid pervious material that has been treated to be impervious, such as impregnated fluid repellent paper or non-woven material, including nonwoven fabric material, or a flexible foam, such as polyurethane or cross-linked polyethylene.

Optionally, the barrier 40 may be breathable, i.e., permits vapor to transpire. Known materials for this purpose include nonwoven materials, monolithic and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

The fluid management articles of the present invention are intended to manage low volumes of fluid encountered both menstrually and intermenstrually, i.e, daily. Preferably, the unitary core structure of the various embodiments of the present invention have less than 0.1 grams of absorbent fibers, and the fluid management article has a total capacity of 6.0 grams or less, as determined by the total capacity test defined in the test methods section,

In addition to fluid capacity, the articles of the present invention are designed to be extremely comfortable and non-obtrusive to a user. The collective design attributes are intended to provide daily confidence without compromise to lifestyle, including activity and clothing. Two variables, which may affect the before mentioned design characteristics, are article caliper, compressibility and flexibility. The articles have a caliper of 2.0 millimeters or less, and preferably 1.5 millimeters or less. Flexibility is measured by a flexural resistance test, described in great detail in the test methods section. Preferably, the articles have a flexural resistance of about 40 grams or less, and more preferably about 35 g or less. Preferably the articles have a compressibility of at least 45% as determined by compressibility test defined in the test methods section.

### Bonding Methods

The layers of the absorbent article may be, but not necessarily, bonded, e.g., glued or adhered, to the adjacent layer. For example, the underside of the cover 20 may be adhered to the topside of the absorbent core 50. The underside of the absorbent core 50 may be adhered to the topside of the barrier layer 40. Any methods known in the art, such as, fusion bonding, adhesive attachment, or by any other securement means can be used to secure the individual layers together to form the final absorbent article. Included within such methods are coembossing, thermobonding, mechanical bonding, and the like. Fusion bonding includes heat bonding, ultrasonic bonding, and the like.

Adhesive is typically used to attach the layers into a single absorbent article. For example, in one embodiment, the body facing cover 10 is attached to the barrier layer 50 with adhesive NW1023 available from H.B Fuller and Company (St. Paul, MN). The adhesive may be applied in any method.

Adhesive may include pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton™ elastomers from Shell Chemical Company, Vector™ elastomers from Dexco, SolpreneTM from Enichem Elastomers and Stereon™ from Firestone Tire & Rubber Co.; hot melt adhesive based on olefin polymers and copolymers where in the olefin polymer is a terpolymer of ethylene and a comonomers, such as vinyl acetate, acrylic acid, methacrylic acid, ethyl acrylate, methyl acrylate, n-butyl acrylate vinyl silane or maleic anhydride. Commercial examples of these types of polymers include Ateva( polymers from AT plastics), Nucrel( polymers from DuPont), Escor (from Exxon Chemical).

Additionally, dots or depressions 60 formed from the co-embossing allow the absorbent article to be rolled into a compact structure without the cover surface wrinkling. Upon unrolling and removal of the release paper, the absorbent article does not retain "memory" of being rolled. In other words, the absorbent article lays flat or conforms to the article to which it is placed on, in this instance, the crotch portion of a garment such as underwear.

The absorbent article of the present invention may be applied to the crotch of a garment by placing the garment-facing surface against the inside surface of the crotch of the garment. Various methods of attaching absorbent articles may be used. For example, chemical means, e.g., adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Adhesive may be applied to the garment-facing side of the absorbent article. The positioning adhesive may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive, or releasable tenacious means. Suitable adhesive compositions, include, for example, water-based pressure-sensitive adhesives, such as acrylate adhesives. Alternatively, the adhesive composition may include rapid setting thermoplastic "hot melt," rubber adhesives, two-sided adhesive tape, and the like.

Where positioning adhesive is used on the garment-facing side of the barrier layer 40, a release strip may be applied to protect the adhesive on the absorbent article prior to attaching the absorbent article to the crotch. The release strip can be formed from any suitable sheet-like material that adheres with sufficient tenacity to the adhesive to remain in place prior to use but which can be readily removed when the absorbent article is to be used. Optionally, a coating may be applied to release strip to improve the ease of removing the release strip from the adhesive. Any coating capable of achieving this result may be used, e.g., silicone. Because typical release strips have memory properties, it is contemplated that while a release strip can be used with the rolled disposable article of the present invention, the memory properties of the disposable absorbent article of the present invention are separate and distinct from any memory properties of the release paper.

### Wings

Wings, also called, among other things, flaps or tabs, may also be part of the absorbent article of the present invention. Wings and their use in sanitary protection articles are described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly.

As disclosed in the above documents, wings are, generally speaking, flexible and configured to be folded over the edges of the underwear so that the wings are disposed between the edges of the underwear.

In addition, there may be one or a plurality of wings incorporated into the present invention. Where opposed wings are present along the longitudinal edges of the absorbent article of the present invention, such wings may be directly opposed or may be offset from the wings located along the opposite longitudinal edge of the absorbent article.

When present, the wings may preferably be folded inwardly prior to rolling the disposable absorbent article.

### Overwrap

The rolled absorbent article can be maintained in a rolled configuration using a wrapper, a ring, a hook and loop system, adhesives and mixtures thereof. For example, a wrapper made of standard cellophane or polypropylene films and including any heat sealable material may be used.

The overwrap can also be embossed (including microembossing), electronically altered (including corona discharge treatments), coated (including vapor deposition, and sputtering), laminated (including film/nonwoven laminate) and the like.

Optionally, the overwrap may be used as a release strip and as such shall coating may be applied to the overwrap to improve the ease of removabilty from the adhesive. Any coating capable of achieving this result may be used, e.g., silicone.

In addition, slip agents such as fluid lubricants or solid layers with a reduced coefficient of friction may be applied to the overwrap package at any appropriate portion of the manufacturing process.

### Miscellaneous

Any or all of the cover, absorbent layer, transfer layer, backsheet layer, and adhesive layers may be colored and/or transparent. Such coloring includes, but is not limited to, white, black, red, yellow, blue, orange, green, violet, and mixtures thereof. Color may be imparted according the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like.

Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49: 1), Red Lake C (Pigment Red), and the like.

Also contemplated herein include asymmetrical and symmetrical articles having parallel longitudinal edges, dog bone- or peanut-shaped, circular, oval and the like.

The silhouette of the disposable fluid management article of the present invention may be configured to be used with conventional underwear or may be configured to conform to thong garments. As used herein, the term thong includes, but is not limited to, thong underwear, thong swimming suit bottom, G-strings, Rio cut underwear, Rio cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself. The fluid management article may include other known materials, layers, and additives, such as, foam, net-like material, perfumes, medicaments or pharmaceutical agents, moisturizers, odor control agents, and the like. The fluid management article can optionally be embossed with decorative designs.

The fluid management article 10 may be made from any of the processes known to one of ordinary skill in the art. For example, a continuous length of cover material may be laminated to a continuous length of absorbent material. This laminate may then be laminated to a backsheet to form a fluid management article. The fluid management article can then be rolled up and over-wrapped to form a single unitary or individual absorbent article. In another embodiment, the cover is laminated to the backsheet and then rolled up and over-wrapped.

In one embodiment of this invention, the fluid management article 10 may be drapeable, that is having a flexural resistance of about 40g. or less as tested by the Modified Circular Bend Test, ASTM 4032-82 and as described in published US patent application no. 20030114822-A1. . In another embodiment, the fluid management article 10 has a cover 20, a backsheet 40, and a flexural resistance of less than 35 g.

Once the absorbent article is unrolled and the release paper removed, it can be placed onto the user's underwear. This particular embodiment conforms to the underwear and is not noticeable to the user.

### Example

Fluid management articles were constructed using the following structures and steps:
a. 50% 3.3d 27 mm (or specific weight) rayon Danufil V from Acordis Kelheim, GmbH is dispersed within a blend of 3d 45 mm bicomponent fibers made of polypropylene and polyethylene is through air bonded to form a 25 gsm composite nonwoven web available from Guang Zhou ES Fiber Co., Ltd. This forms the first layer of the core. The second layer of the core is a 25 gsm composite nonwoven web of hot through air bonded 2d 45 mm PP/PE blend available from Guang Zhou ES Fiber Co., Ltd. The first layer and second layer are bonded together by through air bonding process to form a unitary structure having a final basis weight of 50 gsm. This unitary structure forms the core.
b. The cover is formed from a 30 gsm nonwoven through air bonded web made from 2d 45 mm PP/PE blend. The cover is embossed with a surface pattern such as a flower.
c. The barrier is a microembossed white film, 23 gsm,of polyethylene and polypropylene from - Foshan Huahan Santitary Material Ltd.

The cover and core are first joined together by adhesive HB Fuller NW1023 available from H.B Fuller and Company (St. Paul, MN) and then co-embossed at two stations-the first station being the holes and the second station is the flower embossing.

The cover/core layers are joined to the barrier with adhesive HB Fuller NW 1023 available from H.B Fuller and Company (St. Paul, MN) and the outer perimeter of the article is crimped then cut into the desired silhouettes.

Positioning adhesive HB Fuller 1662x or HB Fuller 1138 is placed on the silicone-coated side of the release film/ overwrap and subsequently transferred to the garment facing surface of the barrier then trifolded.

## Claims

1. A fluid management article (10) comprising
a) a silhouette comprising two opposed end portions (24,26) and two opposed longitudinal edges (32,34) connecting the two opposed end portions (24,26); and
b) a unitary core structure (50) consisting essentially of an integrally formed composite material, the composite material comprising a second material (54) comprising non-absorbent fusible fibers and a first material (52) comprising at least 50% non-absorbent fusible fibers interspersed with absorbent cellulosic based fiber wherein the first and second materials (52, 54) are integrated with the application of heat thereto, and further comprising a cover (20) and a barrier (40), wherein the fluid management article (10) has a caliper of 2.0 mm or less.

2. A fluid management article (10) of claim 1, wherein the unitary core structure (50) contains less than 0.1 grams absorbent cellulosic based fibers.

3. A fluid management article (10) of claim 1, wherein the unitary core structure (50) has a basis weight of about 50 gsm.

4. A fluid management article (10) of any of claims 1 to 3, wherein the cover (20) and unitary core structure (50) are laminated together to form a laminated structure, preferably by adhesive, embossing, or combinations thereof.

5. A fluid management article (10) of claim 5, wherein said nonabsorbent fusible fibers in said first material (52) and said nonabsorbent fibers in said second material (54) comprise at least 84% by weight of said fluid management article (10).

6. A fluid management article (10) of any of claims 1 to 5, having a caliper of less than 1.5 mm.

7. A fluid management article (10) of any of claims 1 to 6 having a flexural resistance of 40 g or less.

## Patentansprüche

1. Gegenstand (10) für die Handhabung von Fluiden, der aufweist
a) einen Umriss, der zwei gegenüberstehende Endbereiche (24, 26) und zwei gegenüberstehende längsverlaufende Kanten (32, 34), die die beiden gegenüberstehenden Endbereiche (24, 26) verbinden, aufweist; und
b) ein einheitliche Kernstruktur (50), die im Wesentlichen aus einem einstückig gebildeten Verbundmaterial besteht, wobei das Verbundmaterial ein zweites Material (54), das nicht absorbierende schmelzbare Fasern aufweist, und ein erstes Material (52), das wenigstens 50 % nicht absorbierende schmelzbare Fasern, in die absorbierende zellulosisch basierte Faser eingemischt ist, aufweist, umfasst, wobei das erste und das zweite Material (52, 54) durch die Anwendung von Wärme auf diese integriert werden, und der weiter eine Abdeckung (20) und eine Endmaß von 2.0 mm oder weniger hat.

2. Gegenstand (10) für die Handhabung von Fluiden nach Anspruch 1, bei dem die einheitliche Kernstruktur (50) weniger als 0.1 Gramm absorbierende zellulosisch basierte Fasern enthält.

3. Gegenstand (10) für die Handhabung von Fluiden nach Anspruch 1, bei dem die einheitliche Kernstruktur (50) ein Riesgewicht von ungefähr 50 g/m² hat.

4. Gegenstand (10) für die Handhabung von Fluiden nach einem der Ansprüche 1 bis 3, bei dem die Abdeckung (20) und die einheitliche Kernstruktur (50) aneinander laminiert sind, um eine laminierte Struktur zu bilden, bevorzugt durch Klebmittel, Prägen oder Kombinationen aus diesen.

5. Gegenstand (10) für die Handhabung von Fluiden nach Anspruch 5, bei dem die nicht absorbierenden schmelzbaren Fasern in dem ersten Material (52) und die nicht absorbierenden Fasern in dem zweiten Material (54) wenigstens 84 Gew.-% des Gegenstandes (10) für die Handhabung von Fluiden umfassen.

6. Gegenstand (10) für die Handhabung von Fluiden nach einem der Ansprüche 1 bis 5, der ein Endmaß von weniger als 1.5 mm hat.

7. Gegenstand (10) für die Handhabung von Fluiden nach einem der Ansprüche 1 bis 6 mit einem Biegewiderstand von 40 g oder darunter.

## Revendications

1. Article de gestion de fluide (10) comprenant
a) une silhouette comprenant deux portions d'extrémité opposées (24, 26) et deux bords longitudinaux opposés (32, 34) reliant les deux portions d'extrémité opposées (24, 26) ; et
b) une structure de noyau unitaire (50) consistant essentiellement en un matériau composite solidairement formé, le matériau composite comprenant un second matériau (54) comprenant des fibres fusibles non absorbantes et un premier matériau (52) comprenant au moins 50 % de fibres fusibles non absorbantes intercalées avec une fibre cellulosique absorbante où les premier et second matériaux (52, 54) sont intégrés avec l'application de chaleur à ceux-ci, et comprenant en outre une couverture (20) et une barrière (40), où l'article de gestion de fluide (10) a un calibre de 2,0 mm ou moins.

2. Article de gestion de fluide (10) selon la revendication 1, dans lequel la structure de noyau unitaire (50) contient moins de 0,1 gramme de fibres cellulosiques absorbantes.

3. Article de gestion de fluide (10) selon la revendication 1, dans lequel la structure de noyau unitaire (50) a un grammage d'environ 50 g/m²_{.}

4. Article de gestion de fluide (10) selon l'une quelconque des revendications 1 à 3, dans lequel la couverture (20) et la structure de noyau unitaire (50) sont stratifiées ensemble pour former une structure stratifiée, de préférence par adhésif, embossage ou leurs combinaisons.

5. Article de gestion de fluide (10) selon la revendication 5, dans lequel lesdites fibres fusibles non absorbantes dans ledit premier matériau (52) et lesdites fibres non absorbantes dans ledit second matériau (54) comprennent au moins 84 % en poids dudit article de gestion de fluide (10).

6. Article de gestion de fluide (10) selon l'une quelconque des revendications 1 à 5, ayant un calibre inférieur à 1,5 mm.

7. Article de gestion de fluide (10) selon l'une quelconque des revendications 1 à 6 ayant une résistance à la flexion de 40 g ou moins.
